**Europäisches Patentamt**

**European Patent Office**

**Office européen des brevets**

(19)

(11) Veröffentlichungsnummer: **0 021 257**
**B1**

(12) **EUROPÄISCHE PATENTSCHRIFT**

(45) Veröffentlichungstag der Patentschrift: **26.10.83**

(21) Anmeldenummer: **80103261.6**

(22) Anmeldetag: **12.06.80**

(51) Int. Cl.³: **C 12 N 5/00, C 12 M 3/00, C 12 P 21/00**

(54) **Verfahren zur Oberflächenzüchtung kernhaltíger Zellen und Gewinnung von Zellkultur-abhängigen Stoffen.**

(30) Prioritat: **28.06.79 DE 2926091**
**17.04.80 DE 3014814**

(43) Veröffentlichungstag der Anmeldung:
**07.01.81 Patentblatt 81/1**

(45) Bekanntmachung des Hinweises auf die Patenterteilung:
**26.10.83 Patentblatt 83/43**

(84) Benannte Vertragsstaaten:
**AT BE CH DE FR GB IT LI LU NL SE**

(56) Entgegenhaltungen:
**CH - A - 525 969**
**DE - A - 2 031 768**
**DE - A - 2 300 567**
**DE - A - 2 320 885**
**FR - A - 1 598 245**
**FR - A - 2 311 845**
**US - A - 3 740 321**

(73) Patentinhaber: **Dr. Karl Thomae GmbH**
**Postfach 1755**
**D-7950 Biberach (Riss) (DE)**

(72) Erfinder: **Merk, Walter, Dr.**
**Oberer Bühl 12**
**D-7950 Biberach 1 (DE)**

Courier Press, Leamington Spa, England.

Verfahren zur Oberflächenzüchtung kernhaltiger Zellen und Gewinnung von Zellkultur-abhängigen Stoffen

Es ist bekannt, daß die Züchtung von nicht transformierten, kernhaltigen Zellen (Eukaryonten) nur mit der Oberflächen-Züchtungsmethode gelingt. Dies bedeutet, daß die in Suspension gehaltenen Zellen nicht wachsen. Sie müssen vielmehr Gelegenheit haben, sich auf einer geeigneten Oberfläche abzusetzen, um sich dort bis zur Kontakt-Inhibition vermehren zu können.

Die Oberflächen-Züchtungsmethode wird in ihrer einfachsten Art in einem Gefäß mit planem Boden durchgeführt, wobei darauf geachtet werden muß, daß der Gefäßboden möglichst genau waagerecht liegt. Ein derartiges Gefäß besteht vorzugsweise aus Glas oder einem geeigneten Kunststoff, wobei die Größe des Behälters letztlich nur durch die erforderliche Handhabung beschränkt wird. Um hierbei eine möglichst große Oberfläche zu erzielen, existieren auch Behälter, in denen mehrere horizontal übereinander liegende plane Flächen, welche durch entsprechende Öffnungen miteinander verbunden sind, angeordnet sind. Dem gleichen Prinzip folgend wurde auch ein Wannenstapler beschrieben, der 10 übereinanderliegende plane Kulturflächen mit je 600 cm² Oberfläche aufweist, wobei dieser Behälter auch zu Blöcken à 4 Stück parallel geschaltet werden kann.

Desweiteren wird bei anderen Zellkultur-Verfahren durch Drehen des Behälters die gesamte Innenfläche, z.B. bei den sogenannten Rollerflaschen, für die Zellkultur verwendet. Eine derartige Rollerflasche kann mit Scheiben oder anderen Körpern so beschickt werden, daß die Oberfläche vergrößert und außerdem die Versorgung mit Nährstoffen mittels eines Mediums sowie das erforderliche Sauerstoffangebot sichergestellt werden kann.

Diese Verfahren finden jedoch dort ihre Grenzen, wo sie wegen des Behälterumfanges nicht mehr oder nur schwer gehandhabt werden können. Überdies lassen sich während des Betriebs die für Änderungen in der Kultur erforderlichen Schlauchverbindungen wegen der Bewegung nur schwer bewerkstelligen.

Ferner wird in der FR—A—1.598.245 ganz allgemein ein Oberflächenzüchtungsverfahren unter Benützung von beweglichen Füllkörpern geoffenbart. In dieser Patentschrift, welche kein einziges Beispiel enthält, werden lediglich als bevorzugte Oberflächenkörper Kugeln mit einem Durchmesser von einigen mm und Raschig-Ringe ohne jegliche weiteren Angaben erwähnt, außerdem wird als einziger Wirkstoff für die Füllkörper Glas genannt.

Es gibt jedoch auch Geräte, die aus einem Behälter mit mehreren Zellkulturflächen und einer Pumpe bestehen. Durch Umpumpen des Mediums wird das erforderliche Nährstoff- und Sauerstoffangebot gewährleistet. So wird beispielsweise ein Gerät von 2 Liter Volumeninhalt beschrieben, in dem die planen Kulturflächen durch kleine Glas-Zylinder ersetzt werden und der pH des umgepumpten Mediums durch Begasung reguliert wird. In gleicher Weise wird bei der bekannten Organ-Perfusions-Technik verfahren.

Bei dem beschriebenen Oberflächen-Züchtungsverfahren mit kleinen Glaszylinder von ca. 6 mm Länge, ca. 4 mm äußerem Durchmesser und ca. 2 mm innerem Durchmesser in einem Behälter bei gleichzeitigem Umpumpen des Mediums ist jedoch aufgrund der auftretenden physikalischen Wirkungen wie Benetzung, Kappilarwirkung und anderer Grenzflächenwirkungen insbesondere wegen der Bildung von Luftblasen eine Bedeckung aller Kulturflächen nicht möglich.

Aus den gleichen Gründen lassen sich bei den bisher bekannten Verfahren die mit Zellen bedeckten Kulturflächen nicht ausreichend spülen. Es erfolgt kein vollständiger Rückfluß, da ein Teil des ursprünglichen Mediums hartnäckig festgehalten wird. Die physikalischen Kräfte der bisher bei der Oberflächenvermehrung benützten Glas-Zylinder oder Kugeln verhindern aber gerade beispielsweise bei der Fibroblasten-Interferon-Herstellung die erforderliche vollständige Entfernung des Induktionsmediums bzw. des Fremdproteins mittels Spülen. Außerdem zerstören die bei der Interferonherstellung üblicherweise verwendeten Pumpen, bei welchen Scherkräfte, auftreten, größtenteils das bereits hergestellte Interferon.

Unter der Bezeichnung Microcarrier-Technik werden geeignete Kugeln, wie beispielsweise aus Dextran, mit einem Durchmesser von ca. 0,15—0,25 mm im Medium suspendiert und durch Bewegung in Suspension gehalten. Auf der Oberfläche dieser Kugeln lassen sich bei geeigneter Technik (siehe Biotechnology and Bioengineering, Vol. XXI, 433—442 (1979)) Zellen züchten. Die zur Verfügung stehende Oberfläche gegeben durch die Beziehung Medium/Volumen, ist hier besonders groß. Sie beträgt bei 0,5 g Dextran-Trägern in 100 ml Medium 3.000 cm². Die mit Zellen bewachsenen Kugeln sind jedoch gegen mechanische Einflüsse sehr empfindlich. Durch Absetzenlassen der Carrier und Absaugen der überstehenden Flüssigkeit wird das Medium entfernt. Damit läßt sich gleichfalls keine vollständige Spülung ermöglichen.

Überraschenderweise wurde nun gefunden, daß sich die oben geschilderten Nachteile bei der Oberflächenzüchtung und somit bei der Gewinnung von Zellkultur-abhängigen Stoffen wird Interferon, Viren, Fermente, Immunabwehrstoffe wie z.B. Immunglobuline ausschalten lassen, wenn als Kulturflächen Körper verwendet werden die auf Grund ihrer Form keine störenden Grenzflächenwirkungen erzeugen und somit eine vollständige Bedeckung und ausreichendes Spülen der Kulturfläche gestattet. Zweckmäßigerweise werden hierbei Füllkörper verwendet, die eine für eine Zellkultur günstige Oberfläche aufweisen und ihnen eine ausreichende Haftungsmöglichkeit bieten, sowie nach Auslaufen des

2

**0 021 257**

Mediums weniger als 8% des Mediums bezogen auf freies Volumen zurückhalten und bei denen gleichzeitig das Verhältnis Oberfläche der Füllkörper zum freien Volumen 20:1 bis 5:1 cm$^{-1}$ beträgt.

Als derartige Füllkörper kommen Wendeln, Sattelkörper, Spiralen oder Drahtspiralen jeweils aus Metall, z.B. aus Titan, $V_2$A- oder $V_4$A-Stahl, in Betracht.

Besonders bevorzugte bewegliche Füllkörper stellen jedoch die Spiralen, Wendeln und Sattelkörper dar, insbesondere Wendeln und Drahtspiralen in gestreckter form aus $V_2$A- oder $V_4$A-Stahl, bei denen sich die einzelnen Windungen gegenseitig nicht berühren, oder Sättel, da diese gleichzeitig bei einer großen Oberfläche auf das umlaufende Medium nur einen geringen Fließwiderstand und beim Spülen nur eine geringe Haftwirkung auf das Medium aufweisen. Als Drahtspiralen sind hierbei insbesondere diejenigen geeignet, die eine Gesamtlänge von 4—8 mm, vorzugsweise jedoch 5—7 mm, eine Gesamtbreite von 4—8 mm, vorzugsweise jedoch 5—7 mm, und einen Drahtdurchmesser von 0,4—0,8 mm, vorzugsweise jedoch 0,5—0,7 mm, aufweisen, wobei der Zwischenraum der einzelnen Windungen 0,1—0,5 mm, vorzugsweise, jedoch 0,2—0,4 mm, beträgt.

Beispielsweise wurden die Füllkörper

A = $V_4$A-Spiralen (äußerer Durchmesser: 6 mm, Drahtdurchmesser: 0,6 mm, Länge: 6 mm) und
B = Sättel (Durchmesser: 6 mm, Länge: 6 mm)
im Vergleich zu
C = Glaszylinder (äußerer Durchmesser: 4 mm, innerer Durchmesser: 2 mm, Länge: 6 mm)
wie folgt auf ihre Eignung bei dem erfindungsgemäßen Verfahren untersucht:

*Methodik:*

1. Ein Glasrohr (innerer Durchmesser: 3—5 cm) wird unten zu einem Trichter verjüngt, mit einem Rohr von ca. 0,5 cm innerer Durchmesser als Auslauf angestückt und mit einem Hahn versehen. Am Übergang des Trichters zum 0,5 cm Rohr wird eine Markierung angebracht. Dann wird bei geschlossenem Hahn das Glasrohr bis zu dieser Marke mit Wasser gefüllt und das Volumen durch Wiegen des Auslaufs bestimmt. Anschließend wird wieder bis zu dieser Marke mit Wasser gefüllt und eine abgewogene Menge Wasser (z.B. 200 ml) zugegeben. Der obere Spiegel wird mit einer Marke versehen. Dann wird das Wasser bis zur unteren Marke abgelassen. Gleichzeitig wird die Restmenge Wasser, die an der Glaswand haften bleibt durch Wiegen der abgelassenen Menge und Subtrahierung von dem angesetzten Sollvolumen ermittelt. Das Ganze wird mehrmals wiederholt und der Mittelwert als Leerwert festgehalten.

Vor jedem Versuch muß das Prüfrohr vollständig trocken sein (z.B. durch Auswaschen mit Aceton und anschließendem Durchblasen mit Luft). Ebenso sind die zu prüfenden Füllkörper im Trockenschrank zu trocknen. Zur Prüfung werden die Füllkörper in das Glasrohr bis zur oberen Marke eingefüllt. Dabei wird das Gewicht der eingefüllten Füllkörper und die Stückzahl ermittelt. Anschließend wird bei geschlossenem Hahn bis zur oberen Marke eine abgewogene Menge Wasser eingefüllt, die Einfüllmenge durch Wiegen der übrigbleibenden Menge ermittelt und als ''freies Volumen'' festgehalten. Anschließend wird der Hahn geöffnet, das bis zur unteren Marke ablaufende Wasser durch Auffangen in ein tariertes Gefäß auf einer Waage ermittelt. Durch Subtraktion der abgelaufenden Menge von ''freien Volumen'' wird die ''Restmenge'' Wasser berechnet, die in den Füllkörpern verblieben ist. Dabei wird der Leerwert berücksichtigt. Die Oberfläche der Füllkörper wird entweder berechnet oder den Angaben des Herstellers entnommen.

Als Ergebnis werden die experimentell ermittelten Daten zu folgenden Werten berechnet: Freies Volumen pro Liter in cm$^3$; Verhältnis der Füllkörper zum freien Volumen und Restmenge nach Auslauf in % zum freien Volumen.

2. Mit dem gleichen Glasrohr (trocken) wird die gleiche Menge Füllkörper eingefüllt und mit gefärbtem Wasser (z.B. 1% Neutralrot $\triangleq$ 100%) aufgefüllt. Dann wird die obere Öffnung luftdicht mit einem Schlauch verschlossen, der mit Wasser gefüllt und durch eine Schlauchpumpe abgeklemmt ist. Nach Öffnung des Hahnes wird über die Schlauchpumpe Wasser in einer abgemessenen Menge von 100 ml pro Minute zugegeben und der Spüleffekt pro Minute durch Messung der Farbintensität im Photometer erfaßt. Der gemessene Wert wird in Beziehung zum Ausgangswert gesetzt und als Spülwirkung in % Restfarbe nach x-facher Spülung mit freier Volumenmenge errechnet. 5 Minuten nach Beginn der Spülung wird der Wasserzulauf abgestellt, die Säule kurz geschüttelt und der Wert nach 30 Sekunden langem Zupumpen gemessen. Der Wert wird als Restfarbe in % nach x-facher Spülung mit freier Volumenmenge nach anschließendem Schütteln angegeben.

Die nachfolgende Tabelle enthälle die gefundenen Werte:

| Füllkörper | Freies Volumen pro Liter in cm³ | Verhältnis der Restmenge nach Oberfläche der Auslauf in % | | Spülwirkung nach x-facher Spülung mit freier Volumenmenge % | | | Restfarbe facher Spülung mit freiem | |
|---|---|---|---|---|---|---|---|---|
| | | Füllkörper zum freien Volumen cm⁻¹ | zum freien Volumen | 2 mal | 3 mal | 4 mal | % | Volumen |
| A | 856 | 10,9 : 1 | 3,05 | 0,26 | 0 | 0 | 0 | 3 |
| B | 622 | 15,4 : 1 | 6,68 | 0,91 | 0,22 | 0 | 0 | 3,6 |
| C | 532 | 21,4 : 1 | 8,95 | 0,99 | 0,37 | 0,18 | 1,25 | 4,1 |

**0 021 257**

Auf Basis der obigen Methodik sind somit alle Füllkörper für das erfindungsgemäße Verfahren geeignet, welche

1. nach Auslaufen des Mediums weniger als 8% des Mediums bezogen auf freies Volumen (Haftinhalt) zurückhalten und

2. nach 4-maligem Spülen mit freier Volumenmenge eine Restfarbe von max. 0,15% bzw. nach 4-maligem Spülen mit freier Volumenmenge eine Restfarbe von max. 0,5% aufweisen.

Derartige Füllkörper ermöglichen ein Anhaften der in Suspension eingebrachten kernhaltigen Zellen, z.B. Fibroblasten-Zellen, Epithelzellen, innerhalb einer kurzen Zeitspanne, beispielsweise während 1 bis 3 Stunden. Desweiteren werden die Nährstoffe des umlaufenden Mediums gut aufgenommen, sodaß ein rasches Wachstum der Zellen gewährleistet ist. Besonders vorteilhaft ist jedoch, daß sich die dicht gewachsenen Kulturen mit Induktionssubstanzen innerhalb des gewünschten Zeitraumes in Verbindung bringen lassen und anschließend die erforderliche Entfernung der Induktionssubstanzen durch einfaches Spülen keinerlei Schwierigkeiten bereitet. Das Spülen kann hierbei kontinuierlich oder diskontinuierlich erfolgen, das heißt durch Ersatz des umlaufenden Mediums durch ein Spülmedium oder durch gegebenenfalls mehrmaliges Ablaufenlassen des Mediums und anschließendem Auffüllen mit einem Spülmedium, in letzterem Fall erfolgt das Einbringen eines Spülmediums vorzugsweise von unten her.

Ferner lassen sich die benützten Füllkörper ohne Schwierigkeiten säubern und anschließend wieder verwenden. Dies bedeutet aber, daß die Zellen auf der Oberfläche derartiger Füllkörper ausreichend haften, um ein ungestörtes Zellwachstum zu gewährleisten, andererseits haften diese aber nicht so fest, daß sich die Zellen bei der anschließenden Säuberung nicht mehr ablösen lassen.

Gegenstand der vorliegenden Erfindung ist somit ein neues Verfahren zur Oberflächenzüchtung von kernhaltigen Zellen (Eukaryonten) und Gewinnung von Zellkultur-abhängigen Stoffen unter Verwendung von Füllkörpern, welche nach Auslaufen des Mediums weniger als 8% des Mediums bezogen auf freies Volumen zurückhalten, also einen geringeren Haftinhalt als 8% aufweisen, und bei denen gleichzeitig das Verhältnis Oberfläche der Füllkörper zum freien Volumen 20:1 bis 5:1 cm$^{-1}$ beträgt. Diese weisen somit keine störenden physikalischen Eigenschaften, z.B. keine störenden Grenzflächenwirkungen (Benetzung, Kapillarwirkung), auf. Zum Umpumpen des Mediums werden zweckmäßigerweise Pumpen verwendet, bei denen keine Scherkräft auftreten, z.B. Schlauchpumpen.

Als besonders vorteilhaft hat sich das erfindungsgemäße Verfahren bei der Fibroblasten-Interferon-Herstellung erwiesen. Hierzu wird ein Reaktionsgefäß, vorzugsweise ein druckstabiles Reaktionsgefäß, z.B. ein 25 l-Doppelmantel-Reaktionsgefäß aus Duranglas, das üblicherweise mit einem Zu- und Ablauf, pH-Elektroden und Permeator versehen ist, mit sorgfältig gewaschenen Füllkörpern, vorzugsweise mit V$_4$A-Drahtspiralen gestreckter Form (Länge: 6 mm, Breite: 6 mm, Drahtdurchmesser: 0,6 mm, Ganghöhe: 0,3 mm) zu ca. 3/4 gefüllt und vorzugsweise mit gespanntem Dampf sterilisiert. Hierbei beträgt das Verhältnis Oberfläche der Füllkörper zum freien Volumen ca. 11/1 cm$^{-1}$. Anschließend werden Zellen, die vorzugsweise durch Trypsinierung von Oberflächen-Zellkulturen gewonnen wurden, in Minimum Essential Medium, dem zweckmäßigerweise 10% fötales Kalbserum und ein Antibioticum wie Kanamycin in einer Konzentration von 200 $\mu$g/ml beigefügt sind, in einer Zelldichte von zweckmäßigerweise 20.000 Zellen/cm$^2$ suspendiert.

Die eingebrachte Zellkultursuspension bleibt ohne Umpumpen 2—4 Stunden bei 37°C, anschließend wird vorzugsweise mit einer Schlauchpumpe bei einer Umdrehungszahl von 30 Umdrehungen pro Minute das Medium umgepumpt und der pH zwischen 7,3 und 7,5 eingestellt. 24—28 Stunden nach Einbringen der Zellkultursuspension wird in regelmäßigen Abständen Medium entnommen und frisches Wachstumsmedium der gleichen Zusammensetzung zugegeben, insgesamt werden in den folgenden 3—4 Tagen 80 Liter Medium zu- und abgeführt. Das weitere Verfahren richtet sich nach dem Stoff, der aus der Zellkultur gewonnen werden soll. Bei Interferon zum Beispiel wird 8—10 Tage nach Ansatz der Zellkultur das gesamte Medium entfernt und anschließend nach bekannten Verfahren, z.B. mit Poly I:C, Mitomycin C, Virus oder anderen geeigneten Substanzen, induziert. Danach wird das gesamte Induktionsmedium entfernt und die Zellen mit Minimum Essential Medium mehrmals gewaschen. Anschließend gibt man erneut Medium zu, dem ein für Interferon geeigneten Stabilisator, z.B. Serum-Albumin, zugefügt war. Diese Flüssigkeit wird erneut wie oben angegeben umgepumpt. Nach weiteren 12—24 Stunden wird das Medium geerntet und das erhaltene Rohinterferon nach an sich bekannten Verfahren konzentriert und gereinigt.

Erfindungsgemäß wird vorzugsweise eine Schlauchpumpe benützt, die bei einer Umdrehungszahl von 25 bis 60 pro Minute eine Förderleistung von 50 bis 400% des Gesamtvolumens pro Stunde aufweist. Das erfindungsgemäße Verfahren läßt sich somit mit erheblich größeren Volumen-Mengen als die bisher bekannten Verfahren durchführen und wird nur durch eine ausreichende Nährstoff- und Sauerstoffversorgung, sowie einer Konstanzhaltung des pH innerhalb eines für die Zellen geeigneten Bereichs begrenzt.

Ein weiterer Vorteil des erfindungsgemäßen Verfahrens besteht darin, daß die während der Produktionsphase gebildeten Zellkultur-abhängigen Stoffe wie z.B. Interferon diskontinuierlich oder kontinuierlich aus dem Kreislauf entnommen, durch neues Medium ersetzt, und beispielsweise das so entnommene Interferon auf eine Reinigungs- und Konzentrierungssäulen gebracht werden kann.

Außerdem bereitet die Zugabe einer sogenannten Stimulatorsubstanz oder eines Gemisches

5

derartiger Substanzen zu jedem gewünschten Zeitpunkt des Verfahrens, um die Ausbeute eines Zellkultur-abhängigen Stoffes zu steigern, bzw. deren Entfernung keinerlei Schwierigkeiten (siehe EP—A—0.005.476).

Das nachfolgende Beispiel soll die Erfindung näher erläutern:

### Beispiel

Ein 25 Liter-Doppelmantel-Reaktionsgefäß aus Duranglas wird mit 17 Litern sorgfältig gewaschenen $V_4A$-Drahtspiralen, gestreckte Form, Länge 6 mm, Breite 6 mm, Drahtdurchmesser 0,6 mm, Ganghöhe: 0,3 mm, gefüllt (Verhältnis Oberfläche der Füllkörper zum freien Volumen ($=$ ca. 11/1 cm$^{-1}$). Zu- und Ablauf werden mit Silicon-Gummischläuchen, Innendurchmesser 8—10 mm, versehen, über pH-Elektroden mit pH-Meter und Permeator geführt und zum Kreislauf verbunden. Die Anlage wird mit gespanntem Dampf sterilisiert. Durch Trypsinierung von Oberflächen-Zellkulturen gewonnene Zellen werden in 17 Liter Minimum Essential Medium mit 10% fötalem Kälberserum und 200 $\mu$g/ml Kanamycin in einer Zahl von 20.000 Zellen/cm$^2$ suspendiert und in das Reaktionsgefäß eingebracht. Das Reaktionsgefäß wird über den Doppelmantel und einem Wasserbadthermostaten auf 37°C aufgeheizt und bei dieser Temperatur geregelt. Die eingebrachte Zellkultursuspension bleibt 2—4 Stunden ohne Umpumpen im Reaktionsgefäß. Anschließend wird der Siliconschlauch in eine Schlauchpumpe eingelegt und bei einer Umdrehungszahl von 30 Umdrehungen pro Minute bei einer Förderleistung von 40—80 l/Stunde umgepumpt. Der pH wird zwischen 7,3 und 7,5 geregelt. 24—48 Stunden nach Ansatz wird in regelmäßigen Abständen Medium entnommen und frisches Wachstumsmedium der gleichen Zusammensetzung zugegeben. Insgesamt werden in den folgenden 3—4 Tagen 80 Liter Medium zu- und abgeführt. 8—10 Tage nach Ansatz der Zellkultur wird das gesamte Medium abgelassen, die Zellen durch Zugabe von Minimum Essential Medium ohne Serum gewaschen und anschließend das Induktionsmedium zugegeben, das 100 $\mu$g/ml Poly I:C und 2,5 $\mu$g/ml Cycloheximid enthält. Das Medium bleibt 3 Stunden im Reaktionsgefäß. Anschließend wird 2 $\mu$g/ml Actinomycin D zugegeben. Eine Stunde nach der Actinomycin D-Zugabe wird das gesamte Induktionsmedium entfernt und die Zellen mindestens 4 × mit Minimum Essential Medium ohne Serum gewaschen. Anschließend wird Minimum Essential Medium mit 1 000 $\mu$g/ml Serum-Albumin zugegeben und, wie vorher beschrieben, umgepumpt. Die pH-Regulierung wird auf 7,5—7,6 eingestellt. 19—20 Stunden später wird das Medium geerntet und nach bekannten Verfahren konzentriert und gereinigt.

Die Ausbeuten an Interferon in der Roh-Lösung betragen unter Bezug auf das internationale Referenz-Präparat G 023—902—527 des National Institutes of Healt (USA) 900—2.000 Einheiten/cm$^2$ Kulturfläche.

Unter Verwendung von verschiedenen Füllkörpern in verschiedenen Ansatzmengen wurde Fibroblasten-Interferon analog mit folgenden Ergebnissen hergestellt:

| Füllkörper | Ansatz-Menge in Liter | Interferon-Referenz-Einheiten/cm$^2$ Kulturfläche |
|---|---|---|
| A | 2,2 | 1.580 |
| A | 17,0 | 1.374 |
| A | 108,0 | 1.103 |
| B | 0,2 | 470 |
| Vergleich (C)* | 0,2 | 23 |
| | 0,2 | 19 |

\* siehe den vorstehend erwähnten Stand der Technik mit mehreren Zellkulturflächen und einer Pumpe

## Patentansprüche

1. Verfahren zur Oberflächenzüchtung von kernhaltigen Zellen und zur Gewinnung von Zellkultur-abhängigen Stoffen aus den so gewonnenen Zellen, dadurch gekennzeichnet, daß als Kulturfläche Wendeln, Sattelkörper, Spiralen oder Drahtspiralen jeweils aus Metall verwendet werden, wobei diese nach Auslaufen des wässrigen Kulturmediums weniger als 8% des Mediums bezogen auf das freie Volumen zurückhalten und gleichzeitig das Verhältnis Oberfläche der Kulturfläche zum freien Volumen zwischen 20:1 und 5:1 cm$^{-1}$ liegt.

2. Verfahren gemäß Anspruch 1, dadurch gekennzeichnet, daß Wendeln, Sattelkörper, Spiralen oder Drahtspiralen aus $V_2$A- oder $V_4$A-Stahl verwendet werden.

3. Verfahren gemäß Anspruch 2, dadurch gekennzeichnet, daß eine Spirale gestreckter Form mit einer Gesamtlänge von 4—8 mm, vorzugsweise jedoch 5—7 mm, einer Gesamtbreite von 4—8 mm, vorzugsweise jedoch 5—7 mm, einem Drahtdurchmesser von 0,4—0,8 mm, vorzugsweise jedoch 0,5—0,7 mm, und einer Ganghöhe von 0,1—0,5 mm, vorzugsweise jedoch 0,2—0,4 mm, oder Sättel je aus $V_2$A- oder $V_4$A-Stahl verwendet werden.

4. Verfahren gemäß Anspruch 3, dadurch gekennzeichnet, daß eine $V_4$A-Drahtspirale benützt wird.

5. Verfahren gemäß den Ansprüchen 1—4, dadurch gekennzeichnet, daß als Zellkulturabhängiger Stoff Interferon hergestellt wird.

**Revendications**

1. Procédé pour la culture superficielle de cellules contenant des noyaux et pour la préparation de substances dépendant de la culture cellulaire à partir des cellules ainsi obtenues, caractérisé en ce qu'on utilise comme surface de culture des hélices, des selles, des spirales ou des boudins, à chaque fois en métal, ceux-ci retenant moins de 8% du milieu par rapport au volume libre après écoulement du milieu de culture aqueux, et en même temps le rapport de la surface de la culture au volume libre étant entre 20:1 et 5:1 cm$^{-1}$.

2. Procédé suivant la revendication 1, caractérisé en ce qu'on utilise des hélices, des selles, des spirales ou des boudins en acier $V_2$A ou $V_4$A.

3. Procédé suivant la revendication 2, caractérisé en ce qu'on utilise une spirale de forme allongée avec une longueur totale de 4—8 mm, mais de préférence de 5—7 mm, une largeur totale de 4—8 mm, mais de préférence de 5—7 mm, un diamètre de fil de 0,4—0,8 mm, mais de préférence de 0,5—0,7 mm, et un pas de 0,1—0,5 mm, mais de préférence de 0,2—0,4 mm, ou des selles en acier $V_2$A ou $V_4$A.

4. Procédé suivant la revendication 3, caractérisé en ce qu'on utilise un boudin d'acier $V_4$A.

5. Procédé suivant les revendications 1—4, caractérisé en ce qu'on prépare de l'interféron comme substance dépendant d'une culture cellulaire.

**Claims**

1. Process for the surface culture of nucleated cells and for the recovery of cell culture-dependent substances from the cells thus obtained, characterised in that coils, saddle-shaped members, spirals or wire spirals each made of metal are used as the culture surface, these objects retaining less than 8% of the medium, based on the free volume, after the aqueous culture medium has run out, and at the same time the ratio of the surface area of the culture surface to the free volume is between 20:1 and 5:1 cm$^{-1}$.

2. Process as claimed in claim 1, characterised in that coils, saddle-shaped members, spirals or wire spirals made of $V_2$A or $V_4$A steel are used.

3. Process as claimed in claim 2, characterised in that a spiral of extended form with a total length of 4—8 mm, preferably 5—7 mm, a total width of 4—8 mm, preferably 5—7 mm, a wire diameter of 0.4—0.8 mm, preferably 0.5—0.7 mm, and a pitch of from 0.1—0.5 mm, preferably 0.2—0.4 mm is used, or saddles each consisting of $V_2$A or $V_4$A steel are used.

4. Process as claimed in claim 3, characterised in that a $V_4$A wire spiral is used.

5. Process as claimed in claims 1 to 4, characterised in that the cell culture-dependent substance produced is interferon.